# EUROPEAN PATENT APPLICATION

(11) **EP 4 293 355 A2**
(43) Date of publication of application: **20.12.2023**
(21) Application number: 22752998.9
(22) Date of filing: 10.02.2022
(51) Int. Cl.: G01N 33/543, G01N 33/545, G01N 33/76, G01N 33/569, G01N 33/58, C09K 11/02, C09K 11/54, C09K 11/56, C08K 9/02, C08K 3/30

(54) **BIOCOMPATIBLE QUANTUM DOT-POLYMER COMPOSITE AND DIAGNOSTIC KIT USING SAME**

(30) Priority: 10.02.2021 KR 20210019364; 08.02.2022 KR 20220016432; 09.02.2022 KR 20220016671
(71) Applicant: Ulsan National Institute of Science and Technology (UNIST), Eonyang-eup Ulju-gun Ulsan 44919 (KR)
(72) Inventor: PARK, Jongnam, Ulju-gun, Ulsan 44919 (KR); KIM, Sunghwan, Ulju-gun, Ulsan 44919 (KR)
(74) Representative: Studio Torta S.p.A.
(86) International application number: PCT/KR2022/002028
(87) International publication number: WO 2022/173238

(57) **Abstract**

The present disclosure relates to a quantum dot-polymer composite, an immunodetection conjugate including the quantum dot-polymer composite, and a diagnostic kit using the same, the composite including a quantum-bead of a 3D self-assembly structure including one or more quantum dots that has a core-shell structure, a surface-modifying ligand arranged on the surface of the quantum dots, and one or more polymer ligands, wherein the one or more quantum dots are dispersed within the quantum-bead, the core including indium phosphide (InP), and the shell that has a thickness of from 3 nm to 9 nm.

## Description

### Technical Field

The present disclosure relates to a biocompatible quantum dot-polymer composite and a diagnostic kit using the same. The present disclosure was funded by the Ministry of Science and Information and Communications Technology under project number 1711112384 titled "Development of next-generation InxGa1-xN quantum dots customized for light-emitting devices".

This application claims priority to Korean Patent Application No. 10-2021-0019364 filed on February 10, 2021, Korean Patent Application No. 10-2022-0016432 filed on February 8, 2022, and Korean Patent Application No. 10-2022-0016671 filed on February 9, 2022, the disclosures of which are hereby incorporated by reference herein in their entirety.

### Background Art

Semiconductor nanoparticles that exhibit optical properties have great potential for applications in bioimaging. Quantum dots (QDs) exhibit fluorescence with a high quantum yield, and their band gap may be tuned by adjusting their size, making it easy to control the wavelength range of light emitted. Since these optical properties show very bright imaging and are stable, it is expected to be a much more effective material than previously used dyes or protein fluorophores for cell imaging.

Typically, gold nanoparticles have been used to diagnose diseases, but since the color change of gold nanoparticles is not large, diagnosis through the color of gold nanoparticles has limitations in increasing sensitivity.

Various studies have been conducted to replace gold nanoparticles with semiconductor nanoparticles, and many studies have reported the use of CdSe-based toxic quantum dots as fluorescent materials to improve the diagnostic sensitivity of in vitro diagnostic kits.

However, since in vitro diagnostic kits require direct handling by human hands, commercialization has been difficult unless toxicity issues are resolved.

Therefore, there is still a need for biocompatible semiconductor nanoparticles, for example quantum dots.

### Disclosure

### Technical Problem

One aspect is to provide a quantum dot-polymer composite including a quantum-bead of a 3D self-assembly structure including one or more quantum dots that has a core-shell structure and one or more polymer ligands, wherein the one or more quantum dots are dispersed within the quantum-bead, wherein the core includes indium phosphide (InP), and wherein the shell has a thickness of 3 nm to 9 nm.

Another aspect is to provide a diagnostic kit using such quantum dot-polymer composites.

Another aspect is to provide an immunodetection conjugate, including a quantum dot-polymer composite and an antibody that specifically binds to a target specimen, that has good biocompatibility and improved detection efficacy.

Another aspect is to provide a detection kit of a target specimen, including the immunodetection conjugate.

Another aspect is to provide a method of detecting a target specimen, including the process of contacting the immunodetection conjugate with a sample including the target specimen.

### Technical Solution

In one aspect, the present disclosure provides a quantum dot-polymer composite including a quantum-bead of a 3D self-assembly structure including one or more quantum dots that has a core-shell structure, a surface-modifying ligand arranged on the surface of the quantum dot, and one or more polymer ligands, wherein the one or more quantum dots are dispersed within the quantum-bead, wherein the core includes indium phosphide (InP), and wherein the shell has a thickness of from about 3 nm to about 9 nm.

According to an embodiment, the shell may have a thickness of about 3 nm to about 8 nm, about 3 nm to about 7 nm, about 3 nm to about 6 nm, about 3 nm to about 5 nm, or about 3 nm to about 4 nm.

According to an embodiment, the shell of the quantum dot may include one or more inorganic particles of zinc telluride (ZnTe), zinc sulfide (ZnS), zinc oxide (ZnO), zinc selenide (ZnSe), zinc selenide sulfide (ZnSeS), indium gallium nitride (InGaN), indium gallium phosphide (InGaP), indium zinc phosphide (InZnP), copper selenide telluride (CuSeTe), copper indium selenide (CuInSe₂), copper indium sulfide (CuInS₂), silver indium sulfide (AgInS₂), and tin telluride (SnTe).

For example, the shell of the quantum dot may include 2 or more different types of inorganic particles. For example, the shell of the quantum dot may include zinc sulfide (ZnS) and zinc selenide (ZnSe).

According to an embodiment, the shell of the quantum dot may include a first shell layer and a second shell layer, wherein the first shell layer may include a first inorganic particle and the second shell layer may include a second inorganic particle. The first inorganic particle and the second inorganic particle may be selected from the inorganic particles exemplified in the aforementioned shells.

For example, the first shell layer may be arranged between the core of the quantum dot and the second shell layer, and a band gap of the second inorganic particle may be larger than a band gap of the first inorganic particle. For example, the band gap of the first inorganic particle and the second inorganic particle may be larger than the band gap of indium phosphide (InP). With such a band gap configuration, electrons and holes may be concentrated from the second inorganic particle through the first inorganic particle to the InP, resulting in improved optical efficiency.

According to an embodiment, when there are two or more quantum dots, the two or more quantum dots may be the same or different.

According to an embodiment, the surface-modifying ligand may include one or more thiol groups. For example, the surface-modifying ligand may be a ligand derived from a linear, branched, or aromatic compound including one or more thiol groups as a functional group.

According to an embodiment, the surface-modifying ligand may be a C₁-C₂₀ alkylthiol group including one or more thiol groups.

According to an embodiment, the polymer ligand may be selected from a random copolymer, a block copolymer, a graft copolymer, or a combination thereof.

According to an embodiment, the polymer ligand may include a hydrophobic portion and a hydrophilic portion.

For example, the hydrophobic portion of the polymer ligand may be aligned toward the center direction of the quantum-bead, and the hydrophilic portion of the polymer ligand may be aligned toward the outside direction of the quantum-bead.

For example, the hydrophilic portion of the polymer ligand may be exposed to the outside of the quantum-bead.

According to an embodiment, the hydrophilic portion may be in the form of a needle or a brush.

According to an embodiment, the polymer ligand may include one or more of an amine group, an amide group, a hydroxy group, a maleimide group, a thiol group, and a carboxylic acid group at an end.

According to an embodiment, the polymer ligand may be one or more selected from polystyrene, polyolefin, polyvinyl acetal, polyester, polyketone, polyether, polyvinyl pyridine, polycarbonate, polydiene, polyacrylic acid, polyamide, and polyimide; their random copolymers and block copolymers; polymers including a poly maleic anhydride group; and graft copolymers including a poly maleic anhydride group and alkylamine.

For example, the polymer including the poly maleic anhydride group may be represented by Formula 1 below, and the graft copolymer including the poly maleic anhydride group and an alkylamine may be represented by Formula 1A or Formula 1B below:

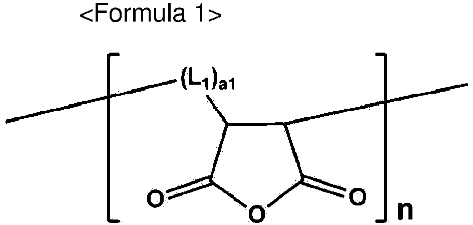

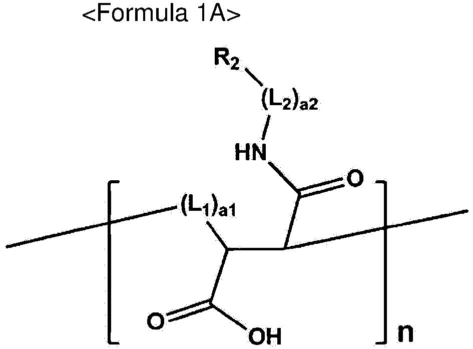

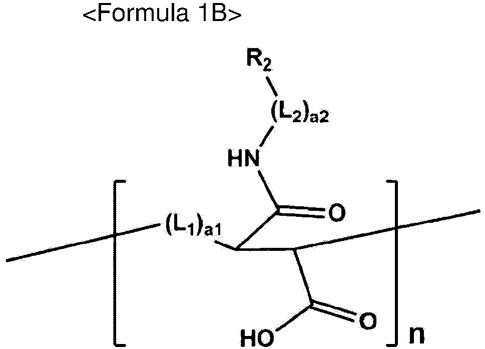

In Formula 1, Formula 1A, and Formula 1B,
L₁ and L₂ are each independently selected from, a C₁-C₂₀ alkyl group or a C₁-C₂₀ alkylene group; and a C₁-C₂₀ alkylene group substituted with one or more selected from deuterium, -F, -Cl, -Br, -I, a cyano group, a nitro group, a C₁-C₁₀ alkyl group, a C₆-C₂₀ aryl group, and a C₁-C₁₀ alkoxy group,
a1 is an integer from 1 to 5,
a2 is an integer from 0 to 5,
R₂ is selected from a C₁-C₂₀ alkyl group; and a C₁-C₂₀ alkyl group substituted with one or more selected from deuterium, -F, -Cl, -Br, -I, a cyano group, a nitro group, a C₁-C₁₀ alkyl group, a C₆-C₂₀ aryl group, and a C₁-C₁₀ alkoxy group,
n is at least 1 but not more than 1000.

According to an embodiment, when there are two or more of the polymer ligands, the two or more polymer ligands may be the same or different.

According to an embodiment, the hydrodynamic diameter (HD) of the quantum-bead may be 50 nm to 1000 nm.

According to an embodiment, the quantum-bead may include one or more antibodies on the surface.

According to an embodiment, the antibodies may be bound to the surface of the polymer ligand.

For example, the bond may be an amide bond.

In another aspect, the present disclosure provides a diagnostic kit including a quantum dot-polymer composite.

For example, the diagnostic kit may use a lateral flow assay (LFA) system.

For example, in the LFA system, the quantum dot-polymer composite may function as a labeling material.

In one aspect, the present disclosure provides an immunodetection conjugate including, a quantum dot-polymer composite; and an antibody conjugated to a surface of the quantum dot-polymer composite and specifically binding to a target specimen,
wherein the quantum dot-polymer composite includes one or more quantum dots that has a core-shell structure, a surface-modifying ligand arranged on the surface of the quantum dots, and a quantum-bead of a 3D self-assembly structure including one or more polymer ligands, wherein the one or more quantum dots are dispersed within the quantum-bead, wherein the core includes indium phosphide (InP), and wherein the shell has a thickness of 3 nm to 9 nm.

According to an embodiment, the shell may have a thickness of about 3 nm to about 8 nm, about 3 nm to about 7 nm, about 3 nm to about 6 nm, about 3 nm to about 5 nm, or about 3 nm to about 4 nm.

According to an embodiment, the shell of the quantum dot may include one or more inorganic particles of zinc telluride (ZnTe), zinc sulfide (ZnS), zinc oxide (ZnO), zinc selenide (ZnSe), zinc selenide sulfide (ZnSeS), indium gallium nitride (InGaN), indium gallium phosphide (InGaP), indium zinc phosphide (InZnP), copper selenide telluride (CuSeTe), copper indium selenide (CuInSe₂), copper indium sulfide (CuInS₂), silver indium sulfide (AgInS₂), and tin telluride (SnTe).

For example, the shell of the quantum dot may include 2 or more different types of inorganic particles. For example, the shell of the quantum dot may include zinc sulfide (ZnS) and zinc selenide (ZnSe).

According to an embodiment, the shell of the quantum dot may include a first shell layer and a second shell layer, wherein the first shell layer may include a first inorganic particle and the second shell layer may include a second inorganic particle. The first inorganic particle and the second inorganic particle may be selected from the inorganic particles exemplified in the aforementioned shells.

For example, the first shell layer may be arranged between the core of the quantum dot and the second shell layer, and a band gap of the second inorganic particle may be larger than a band gap of the first inorganic particle. For example, the band gap of the first inorganic particle and the second inorganic particle may be larger than the band gap of indium phosphide (InP). With such a band gap configuration, electrons and holes may be concentrated from the second inorganic particle through the first inorganic particle to the InP, resulting in improved optical efficiency.

According to an embodiment, when there are two or more quantum dots, the two or more quantum dots may be the same or different.

According to an embodiment, the surface-modifying ligand may include one or more thiol groups. For example, the surface-modifying ligand may be a ligand derived from a linear, branched, or aromatic compound including one or more thiol groups as a functional group.

According to an embodiment, the surface-modifying ligand may be a C₁-C₂₀ alkylthiol group including one or more thiol groups.

According to an embodiment, the polymer ligand may be selected from a random copolymer, a block copolymer, a graft copolymer, or a combination thereof.

According to an embodiment, the polymer ligand may include a hydrophobic portion and a hydrophilic portion.

For example, the hydrophobic portion of the polymer ligand may be aligned toward the center direction of the quantum-bead, and the hydrophilic portion of the polymer ligand may be aligned toward the outside direction of the quantum-bead.

For example, the hydrophilic portion of the polymer ligand may be exposed to the outside of the quantum-bead.

According to an embodiment, the hydrophilic portion may be in the form of a needle or a brush.

According to an embodiment, the polymer ligand may include one or more of an amine group, an amide group, a hydroxy group, a maleimide group, a thiol group, and a carboxylic acid group at an end.

According to an embodiment, the polymer ligand may be one or more selected from polystyrene, polyolefin, polyvinyl acetal, polyester, polyketone, polyether, polyvinyl pyridine, polycarbonate, polydiene, polyacrylic acid, polyamide, and polyimide; their random copolymers and block copolymers; polymers including a poly maleic anhydride group; and graft copolymers including a poly maleic anhydride group and alkylamine.

For example, the polymer including the poly maleic anhydride group may be represented by Formula 1 below, and the graft copolymer including the poly maleic anhydride group and an alkylamine may be represented by Formula 1A or Formula 1B below:

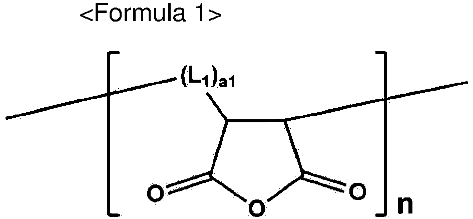

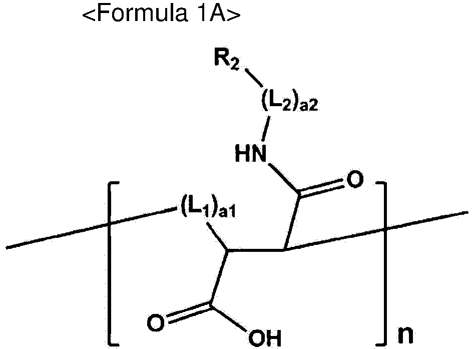

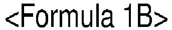

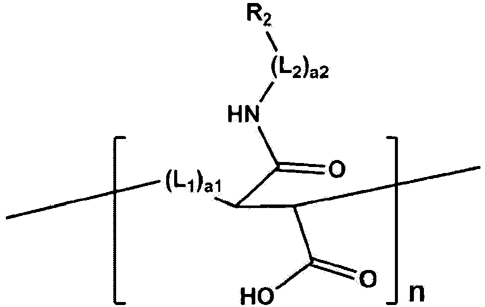

In Formula 1, Formula 1A, and Formula 1B,
L₁ and L₂ are each independently selected from, a C₁-C₂₀ alkyl group or a C₁-C₂₀ alkylene group; and a C₁-C₂₀ alkylene group substituted with one or more selected from deuterium, -F, -Cl, -Br, -I, a cyano group, a nitro group, a C₁-C₁₀ alkyl group, a C₆-C₂₀ aryl group, and a C₁-C₁₀ alkoxy group,
a1 is an integer from 1 to 5,
a2 is an integer from 0 to 5,
R₂ is selected from a C₁-C₂₀ alkyl group; and a C₁-C₂₀ alkyl group substituted with one or more selected from deuterium, -F, -Cl, -Br, -I, a cyano group, a nitro group, a C₁-C₁₀ alkyl group, a C₆-C₂₀ aryl group, and a C₁-C₁₀ alkoxy group,
n is at least 1 but not more than 1000.

According to an embodiment, when there are two or more of the polymer ligands, the two or more polymer ligands may be the same or different.

According to an embodiment, the hydrodynamic diameter (HD) of the quantum-bead may be 50 nm to 1000 nm.

According to an embodiment, an antibody that specifically binds to the target specimen may be conjugated to the surface of the quantum dot-polymer composite via an isopeptide bond between a first peptide tag and a second peptide tag.

For example, the first peptide tag and the second peptide tag may be a pair of SpyTag and SpyCatcher; a pair of SnoopTag or SnoopTagJr and SnoopCatcher; a pair of RrgATag, RrgATag2 or DogTag and RrgACatcher, a pair of IsopepTag and Pilin-C; a pair of IsopepTag-N and Pilin-N; a pair of PsCsTag and PsCsCatcher; and a pair of SnoopTagJr and DogTag mediated by SnoopLigase.

For example, the first tag may include a cysteine (Cys) in its terminal region, and the second tag may include a molecule in its terminal region that binds to the FC region of an antibody that specifically binds to the target specimen.

In another aspect, the present disclosure provides a detection kit for a target specimen including an immunodetection conjugate as described above.

According to an embodiment, the target specimen may be one or more of autoantibodies, ligands, natural extracts, peptides, proteins, metal ions, synthetic drugs, natural drugs, metabolites, genomes, viruses, and substances produced by viruses, and bacteria or substances produced by virus.

According to an embodiment, the kit may be implemented in the form of a lateral flow assay strip.

For example, the lateral flow assay strip may include, a conjugate pad including an immunodetection conjugate as described above, that has a first antibody that specifically binds to a target specimen; a test region immobilized with a second antibody that specifically binds to a target specimen; and a detection portion including a control region immobilized with a third antibody that does not specifically bind to the target specimen but binds to a first antibody that specifically binds to a target specimen in the immunodetection conjugate.

For example, the lateral flow assay strip may further include an absorbent sample pad, wherein the absorbent sample pad, conjugate pad, and detection portion are sequentially arranged in a direction from one end to the other end.

### Advantageous Effects

The quantum dot-polymer composite according to an aspect of the present disclosure is not only biocompatible, but also has excellent photo stability, high brightness and quantum efficiency, and the hydrophilic polymer at one end is capable of binding to antibodies, resulting in excellent antigen-antibody binding, enabling its utilization as a diagnostic kit for disease diagnosis utilizing light from fluorescent materials.

The immunodetection conjugate according to an aspect of the present disclosure includes a quantum dot-polymer composite that has excellent photo stability, high brightness and quantum efficiency, and by adjusting the orientation of the antibody such that the Fab region is exposed to the outside, the detection sensitivity for the target specimen may be significantly improved.

In addition, by varying the type of antibody bound to the quantum dot-polymer composite according to the disease to be detected, early diagnosis of various diseases is possible, and diagnosis can be performed in vitro, and the diagnostic method is easier and faster compared to the typical assay method.

### Description of Drawings

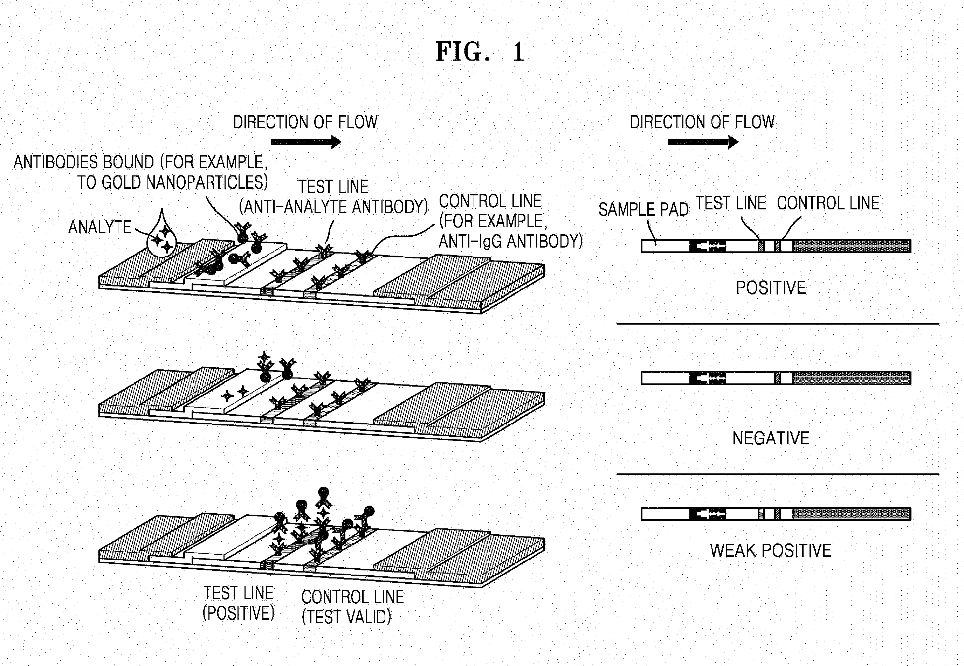

FIG. 1 is a schematic diagram to illustrate the LFA system.
FIG. 2A is a schematic diagram of a quantum dot-polymer composite including a polymer ligand in the form of a brush, FIG. 2B is a schematic diagram of a quantum dot-polymer composite including a polymer ligand in the form of a needle, and FIG. 2C is a schematic diagram showing the high sensitivity of a quantum dot-polymer composite including a polymer ligand in the form of a brush.
FIG. 3A is a schematic diagram illustrating the structure of a block copolymer and a random copolymer, an embodiment of the polymer ligand, and FIG. 3B is a schematic diagram illustrating the structure of a graft copolymer, another embodiment of the polymer ligand.
FIG. 4A is a transmission electron microscopy (TEM) image of a quantum dot synthesized in Synthesis Example 1, and FIG. 4B is a TEM image of a quantum dot synthesized in Synthesis Example 2.
FIG. 5A is a TEM image of a quantum-bead manufactured in Example 1 (left: 200 nm scale; right: 500 nm scale), and FIG. 5B is a TEM image of a quantum-bead manufactured in Comparative Example 1 (left: 200 nm scale; right: 100 nm scale).
FIG. 6A is an image showing the phase conversion of the quantum dots of Synthesis Example 1 and Synthesis Example 2, and the quantum-bead of Example 1 and Example 2, and FIG. 6B is a graph showing the luminous efficiency of the quantum-bead of Example 1 and Example 2 normalized against the luminous efficiency of the quantum dots of Synthesis Example 2.
FIG. 7 is a schematic diagram illustrating the manufacturing process of an immunodetection conjugate in which the orientation of the antibody is adjusted to expose the Fab region to the outside.
FIG. 8A is a graph showing the result of calculating the number of conjugated antibodies per quantum-bead in an immunodetection conjugate according to an embodiment, FIG. 8B is a graph showing the number of activated Fab regions per quantum-bead, and FIG. 8C is a graph showing the access rate (%) to the Fab regions.
FIG. 9 is a schematic diagram illustrating the structure of a lateral flow assay strip according to an embodiment.
FIG. 10 is a schematic diagram schematically illustrating a reaction mechanism in a lateral flow assay strip, according to an embodiment.
FIG. 11 is an image showing the results of analyzing the detection efficacy of a lateral flow assay strip according to an embodiment via fluorescence intensity.

### Mode for Invention

Hereinafter, with reference to the accompanying drawings, various embodiments of the present disclosure are described in detail to facilitate practice by one of ordinary skill in the art to which the present disclosure belongs. The present disclosure may be implemented in many different forms and is not limited to the embodiments described herein.

In order to clearly describe the present disclosure, parts irrelevant to the description are omitted, and identical or similar components are designated by the same reference numerals throughout the specification.

In addition, the size and thickness of each configuration shown in the drawings are arbitrarily indicated for ease of description, and the present disclosure is not necessarily limited to those shown.

In addition, the terms used herein are merely used to describe certain embodiments and are not intended to limit the present inventive ideas. The singular expression includes the plural unless the context clearly indicates otherwise. As used herein, the terms "includes" or "has" are intended to indicate the presence of the features, numbers, processes, actions, components, parts, ingredients, materials, or combinations thereof recited herein, and are not intended to preclude the possibility of the presence or addition of one or more other features, numbers, processes, actions, components, parts, ingredients, materials, or combinations thereof. As used herein, "/" may be construed as "and" or "or" depending on the context.

According to one aspect, provided is a quantum dot-polymer composite including a quantum-bead of a 3D self-assembly structure including one or more quantum dots that has a core-shell structure, a surface-modifying ligand arranged on the surface of the quantum dot and one or more polymer ligands, wherein the one or more quantum dots are dispersed within the quantum-bead, the core includes indium phosphide (InP), and the shell has a thickness of from 3 nm to 9 nm.

As described above, in the case of gold nanoparticles, which have been mainly used as materials for typical in vitro diagnostic kits, there is a problem in where it is difficult to diagnose diseases at an early stage due to poor diagnostic sensitivity. Therefore, attempts have been made to use various types of quantum dot composites as materials for diagnostic kits, but there are limitations in human application due to the inherent properties of quantum dots that are sensitive to moisture and the toxicity of inorganic particles.

The quantum dot-polymer composite according to the present disclosure not only enables application as a diagnostic kit for diagnosing diseases in the human body by using biocompatible quantum dots including biocompatible indium phosphide (InP) as a core and not including elements known to be toxic such as cadmium (Cd), lead (Pb), and the like, but also enables excellent photo stability and quantum efficiency by having a unique structure, thereby maximizing luminescence intensity and antigen-antibody binding power, thereby offering enhanced performance.

As described above, the quantum dot-polymer composite is a 3D self-assembly structure of the quantum dots and polymer ligands. Specifically, the quantum-bead are not in the form of separate magnetic bead, latex bead, or quantum dots bonded to an inorganic or organic support, but have a structure in which quantum dots are uniformly dispersed at a certain distance within the polymer ligand structure, so that the number of quantum dots that may be included in the same volume is large compared to previously developed materials, thereby exerting an effect that may maximize the luminescence intensity.

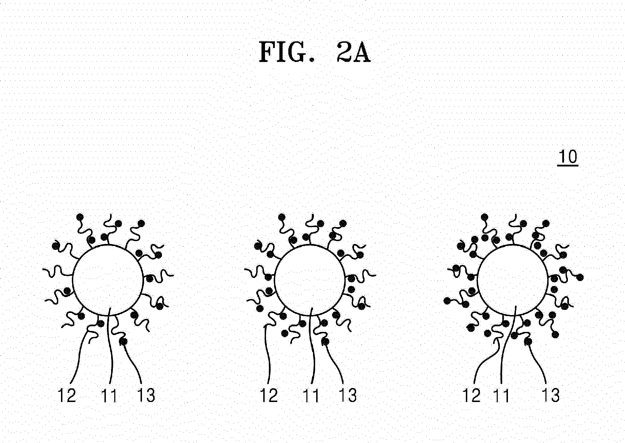

FIG. 2A to FIG. 2B are schematic diagrams of the quantum dot-polymer composite of the present disclosure. Hereinafter, the structure of the quantum dot-polymer composite of the present disclosure will be described in detail with reference to FIG. 2A to FIG. 2B.

Referring to FIG. 2A to FIG. 2B, the quantum dot-polymer composite 10, 20 include quantum-bead in a 3D self-assembly structure including one or more quantum dots and one or more polymer ligands.

Specifically, the quantum-bead includes a core portion 11, 21 of the quantum-bead formed by the quantum dots and a hydrophobic portion of the polymer ligand, and a hydrophilic portion 12, 22 of the polymer ligand as described below. Here, for ease of understanding, the indication of one or more quantum dots included in the quantum-bead and the surface-modifying ligand arranged on the surface of the quantum dot are omitted.

For example, the quantum-bead may be a core-shell structure including a core portion 11, 21 of the quantum-bead formed by the quantum dot and the hydrophobic portion of the polymer ligand and a shell portion including the hydrophilic portion 12, 22 of the polymer ligand.

For example, the quantum dots use nanoparticles of indium phosphide (InP) as the core and shells may include one or more inorganic particles of zinc telluride (ZnTe), zinc sulfide (ZnS), zinc oxide (ZnO), zinc selenide (ZnSe), zinc selenide sulfide (ZnSeS), indium gallium nitride (InGaN), indium gallium phosphide (InGaP), indium zinc phosphide (InZnP), copper selenide telluride (CuSeTe), copper indium selenide (CuInSe₂), copper indium sulfide (CuInS₂), silver indium sulfide (AgInS₂), and tin telluride (SnTe). By not including elements known to be toxic, such as cadmium (Cd) and lead (Pb), the quantum dots are biocompatible and thus can be used as a material for human disease diagnostic kits.

Indium phosphide (InP) inherently has a problem of being easily deteriorated in an external environment such as moisture and oxygen, and also has a limitation in that optical properties are deteriorated during surface treatment to compensate for the instability of InP. Therefore, the utilization of indium phosphide-based quantum dots has been limited.

In an effort to compensate for the instability of InP, core-shell quantum dots are known in which a shell is applied to the surface of an InP core particle. In such InP quantum dots, the degradation of InP by the shell is suppressed, but if the thickness of the shell is increased, the generation of lattice defects, the increase in the amount of defects due to the mismatch of the interlayer crystal structure, or the light scattering or light blocking effect caused by the crystals in the shell where the light emitted by the conversion from the core particles is in the light emission path, may cause a decrease in the optical efficiency, so it is a very important task to properly control the thickness of the shell in the fabrication of quantum dots, and it is generally known that the optical efficiency decreases when the thickness of the shell increases.

In this regard, typically available InP core-shell quantum dots have a shell thickness of about 2 nm ± about 0.5 nm, and it is known that InP quantum dots with this shell thickness have an optical efficiency comparable to that of other quantum dots.

In the process of forming the quantum-bead according to the present disclosure, these typical InP quantum dots not only suffer from defects due to their intrinsic instability, but also from insufficient interaction with the polymer ligand, so that the quantum dots cannot be sufficiently dispersed into the bead, resulting in a decrease in the optical efficiency of the quantum-bead.

In the process of exploring various methods to solve these problems, the inventor of the present disclosure discovered that when the thickness of the InP quantum dot shell is configured from 3 nm to 9 nm, and the surface of the quantum dot is modified with a surface-modifying ligand, for example, a ligand derived from a thiol-based compound including one or more thiol groups, the indium phosphide-based quantum dots are sufficiently included and dispersed within the quantum-bead, and have sufficient luminous efficiency for application in a diagnostic kit, leading to the present disclosure of the present quantum dot-polymer composite.

According to an embodiment, the quantum-bead include one or more quantum dots, wherein when the quantum dots are two or more, the two or more quantum dots may be the same or different.

In addition, the polymer ligand may include a hydrophobic portion (not specifically shown in the drawings) that interacts with the quantum dots to form the center of the quantum-bead, and a hydrophilic portion 12, 22 that aids in aqueous dispersibility and is used for binding to antibody 13, 23 used for application in diagnostic kits.

The polymer ligand may be a random copolymer, a block copolymer, a graft copolymer, or a combination thereof. In this context, a random copolymer is a copolymer in which two or more different types of monomers are randomly arranged. Also, a block copolymer is a copolymer in which two or more different types of polymer chains are connected via chemical bonds.

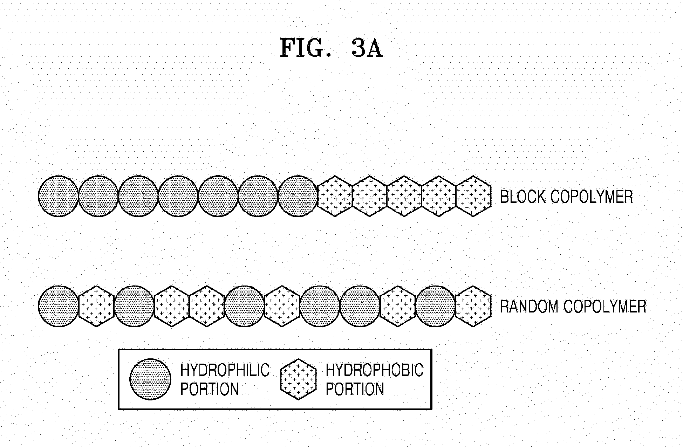

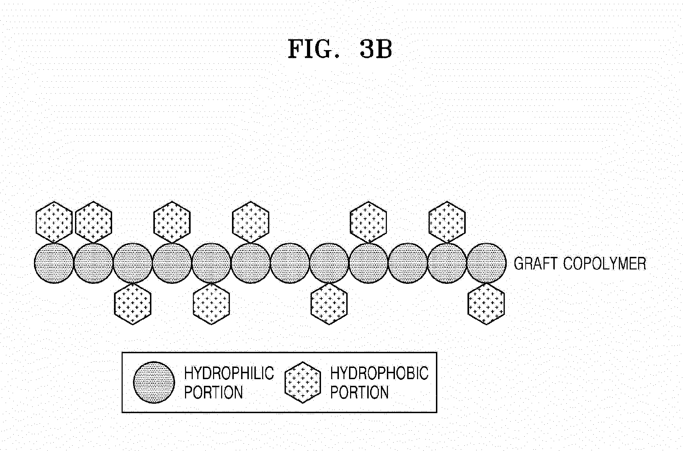

FIG. 3A is a schematic diagram illustrating the structure of a block copolymer and a random copolymer, an embodiment of the polymer ligand, and FIG. 3B is a schematic diagram illustrating the structure of a graft copolymer, another embodiment of the polymer ligand.

First referring to FIG. 3A, the block copolymer is a polymer chain of hydrophilic monomers bonded together and a polymer chain of hydrophobic monomers bonded together, and the random copolymer is a polymerization of hydrophilic monomers and hydrophobic monomers randomly arranged.

Also, referring to FIG. 3B, the graft copolymer is a form in which a previously formed polymer is polymerized with another unit monomer.

Meanwhile, referring again to FIG. 2A to FIG. 2B, the quantum-bead may be formed by hydrophobic interaction of the quantum dots with the hydrophobic portion of the polymer ligand, and aqueous dispersion and binding to the antibody are secured by the hydrophilic portion 12, 22 of the polymer ligand extending to the surface of the core portion 11, 21 of the quantum-bead formed by the quantum dots and the hydrophobic portion of the polymer ligand.

According to an embodiment, the hydrophobic portion (not specifically shown) of the polymer ligands may be aligned toward the center direction of the quantum-bead, and the hydrophilic portion 12, 22 of the polymer ligands may be aligned toward the outside direction of the quantum-bead.

According to an embodiment, the hydrophilic portion 12, 22 of the polymer ligand may have a needle form 22 or a brush form 12.

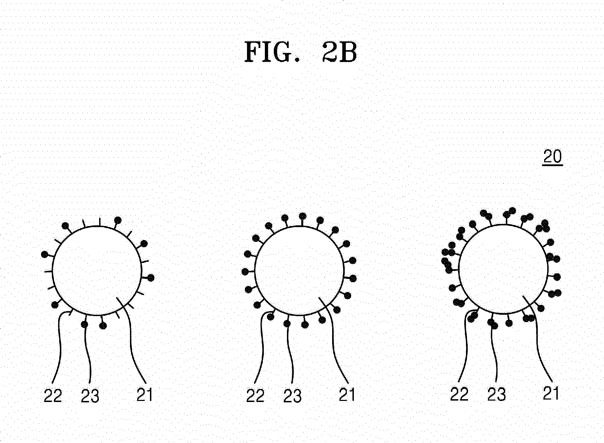

Here, "needle form" refers to the hydrophilic portion 22 of the polymer ligand is generally short and has a single functional group, as shown in FIG. 2B, and "brush form" means that the hydrophilic portion 12 of the polymer ligand is generally long and has several or more functional groups, as shown in FIG. 2A.

For example, although not particularly limited, the hydrophilic portion of the polymer ligand may be in the form of a brush. As described above, when the hydrophilic portion of the polymer ligand is in the form of a brush, the hydrophilic portion of the polymer ligand may bind a greater amount of antibody because the surface region on which the antibody may bind to the hydrophilic portion of the polymer ligand is increased when the antibody binds, as described below, compared to when the hydrophilic portion of the polymer ligand is in the form of a needle.

For example, although not particularly limited, the polymer ligand may be in the form of a brush in the case of a block copolymer, or may be in the form of a needle in the case of a random copolymer and a graft copolymer.

Specifically, referring to FIG. 2A to FIG. 2B, the binding conformation of the antibody 13, 23 in each of the quantum dot-polymer composite 10, 20 when increasing the content of the antibody 13, 23 (increasing antibody content from left to right) may be seen. In the case of the quantum dot-polymer composite 10 of FIG. 2A, which includes the hydrophilic portion 12 of the polymer ligand in the form of a brush, even if the content of the antibody 13 is increased, the antibody 13 bind well to the surface of the hydrophilic portion 12 of the polymer ligand because there is a large surface region for the antibody 13 to bind to the hydrophilic portion 12 of the polymer ligand as described above.

On the other hand, in the case of the quantum dot-polymer composite 20 of FIG. 2B, which includes the hydrophilic portion 22 of the polymer ligand in the form of a needle, when the content of the antibody 23 is increased, there is not enough surface region for the antibody 23 to bind to the hydrophilic portion 22 of the polymer ligand, since the antibody 23 aggregate together rather than on the surface of the hydrophilic portion 22 of the polymer ligand, the antibody binding efficiency may be somewhat poor compared to the hydrophilic portion of the polymer ligand in the brush form.

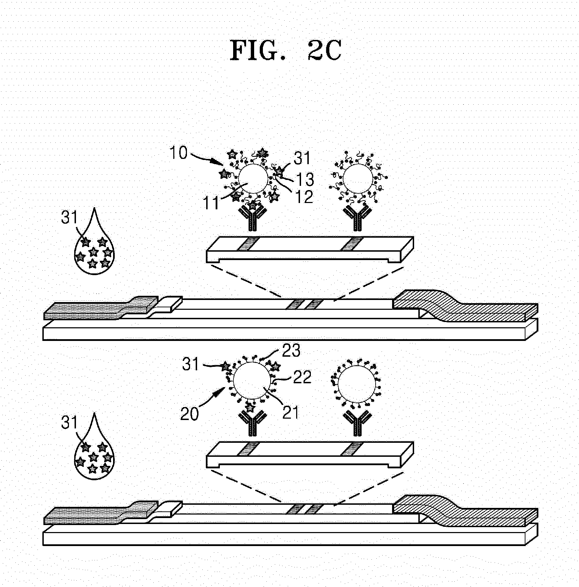

Furthermore, referring to FIG. 2c, it may be seen that when the quantum dot-polymer ligand composite of the present disclosure is applied to the LFA system, the antigen 31 binds more to the antibodies 13, 23 when the hydrophilic portion of the polymer ligand is in the form of a brush (top) compared to when the hydrophilic portion of the polymer ligand is in the form of a needle (bottom).

For example, the polymer ligand may include one or more of an amine group, an amide group, a hydroxy group, a maleimide group, a thiol group, and a carboxylic acid group at an end.

For example, the polymer ligand may be one or more selected from polystyrene, polyolefin, polyvinyl acetal, polyester, polyketone, polyether, polyvinyl pyridine, polycarbonate, polydiene, polyacrylic acid, polyamide, and polyimide; random copolymers and block copolymers thereof; polymers including a poly maleic anhydride group; and graft copolymers including a poly maleic anhydride group and an alkylamine.

For example, the polymer including the poly maleic anhydride group may be one or more selected from and block copolymer; a polymer including the poly maleic anhydride group; and a graft copolymer including the poly maleic anhydride group and an alkylamine.

For example, a polymer including the poly maleic anhydride group may be represented by Formula 1 below, and a graft copolymer including the poly maleic anhydride group and an alkylamine may be represented by Formula 1A or Formula 1B below:

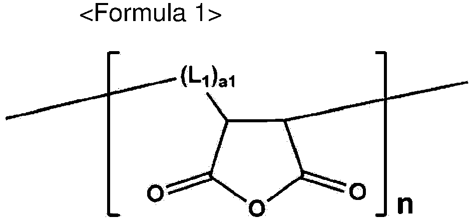

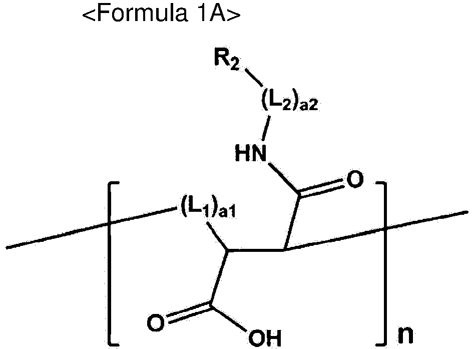

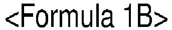

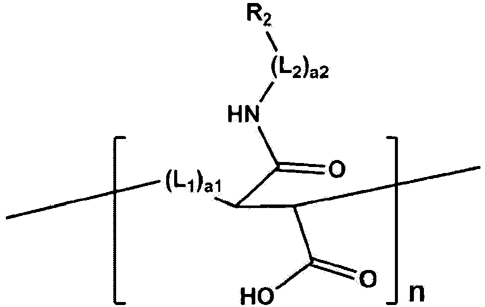

In Formula 1, Formula 1A, and Formula 1B,
L₁ and L₂ are each independently selected from, a C₁-C₂₀ alkylene group; and a C₁-C₂₀ alkylene group substituted with one or more selected from deuterium, -F, -Cl, -Br, -I, a cyano group, a nitro group, a C₁-C₁₀ alkyl group, a C₆-C₂₀ aryl group, and a C₁-C₁₀ alkoxy group,
a1 is an integer from 1 to 5,
a2 is an integer from 0 to 5,
R₂ is selected from a C₁-C₂₀ alkyl group; and a C₁-C₂₀ alkyl group substituted with one or more selected from deuterium, -F, -Cl, -Br, -I, a cyano group, a nitro group, a C₁-C₁₀ alkyl group, a C₆-C₂₀ aryl group, and a C₁-C₁₀ alkoxy group,
n is at least 1 but not more than 1000.

For example, in the above Formula 1A and Formula 1B,
L₁ and L₂ are each independently selected from, methylene group, ethylene group, propylene group, isopropylene group, butylene group, isobutylene group, pentylene group, hexylene group, heptylene group, octylene group, nonylene, and decylene; and
methyl group, ethyl group, propyl group, butyl group, pentyl group, hexyl group, phenyl group, methoxy group, and ethoxy group, substituted with one or more selected from methylene group, ethylene group, propylene group, isopropylene group, butylene group, isobutylene group, pentylene group, hexylene group, heptylene group, octylene group, nonylene, and decylene.

For example, in the above Formula 1A and Formula 1B,
R₂ may be selected from methyl group, ethyl group, propyl group, isopropyl group, butyl group, isobutyl group, pentyl group, hexyl group, heptyl group, octyl group, nonyl group, and decyl group.

For example, the polymer ligand may be a random copolymer of an alkylene maleic anhydride monomer and an alkylamine monomer.

For example, the polymer ligand may be a graft copolymer of a poly alkylene maleic anhydride polymer and an alkylamine monomer.

For example, the polymer ligand may be a poly(isobutylene-alt-maleic anhydride)-hexadecylamine graft copolymer.

Here, where there is two or more polymer ligands, the two or more polymer ligands 12, 22 may be the same or different.

According to an embodiment, the hydrodynamic diameter (HD) of the quantum-bead may be from 50 nm to 1000 nm.

In situations outside of the above range, when the hydrodynamic diameter of the quantum-beads is less than 50 nm, there is a problem of weak luminescence due to the small number of quantum dots included inside, and on the other hand, if the hydrodynamic diameter of the quantum-beads is greater than 1000 nm, there is a problem of poor dispersion of the particles.

For example, the number of quantum dots forming the core portion 11, 21 of the quantum-bead may be appropriately adjusted according to the size of the quantum-bead, although not particularly limited, but may be, for example, 50,000 or less, and may be, for example, 50 to 50,000. For example, the number of quantum dots may be 40,000 or less, for example, may be 30,000 or less, for example, may be 20,000 or less, for example, may be 10,000 or less, for example, may be 5,000 or less, for example, may be 4,000 or less, for example, may be 3,000 or less, for example, may be 1500 or less, for example may be 50 to 1500, for example may be 50 to 1450, for example may be 60 to 1450, for example may be 60 to 1400, for example may be 70 to 1400, for example may be 70 to 1350, for example may be 80 to 1350.

In situations outside of the above range, when the number of quantum dots forming the core portion 11, 21 of the quantum-bead is greater than 1500, emission quenching between neighboring quantum dots may occur, and on the other hand if the number of quantum dots is less than 50, there is a problem of weak luminescence intensity.

The emission wavelength of the quantum-bead is preferably, but not limited to, 450 nm to 700 nm.

According to an embodiment, the antibodies 13, 23 may be bound to the surface of the polymer ligand.

As the antibodies bind to functional groups of the hydrophilic portion 12, 22 of the polymer ligand present on the surface of the quantum-bead, the relative ratio of quantum-beads and antibodies may be adjusted to increase the degree of binding of the antibodies.

For example, the bond may be an amide bond.

In the present disclosure, the antibody is not particularly limited as long as it can bind to the polymer ligand directly or indirectly using a linker, and all types of antibodies may be used. For example, the antibody may be directly bound to the polymer ligand by reacting a reactive amino group of the antibody with a carboxylic acid group of the polymer ligand to form an amide bond, but there is a problem that such binding may not be performed smoothly depending on the structure of the antibody. To address such problems, linkers such as peptides, glutaraldehyde, succinic anhydride, and the like may be used to indirectly bind the antibody to the polymer ligand, and the linkers used are not particularly limited as long as they may mediate the binding between the antibody and the polymer ligand.

According to another aspect of the present disclosure, provided is a diagnostic kit including the quantum dot-polymer composite described above. The use of the diagnostic kit may vary, without limitation, depending on the type of antibody included in the composite described above.

According to an embodiment, the diagnostic kit may be for detecting antigens in vitro. That is, the antigen can be easily detected in vitro, through urine, blood, etc., thereby increasing the speed of diagnosis and enabling easier diagnosis, and simultaneously diagnosing multiple assay samples.

For example, the diagnostic kit may be applied to any in vitro diagnostic kit by modifying the antigen-antibody system.

For example, the diagnostic kit is not particularly limited as long as the antigen-antibody system can be applied to the antigen, but may be one for diagnosing the possibility of pregnancy by detecting HCG hormone, for example, the diagnostic kit can be applied in various ways as a material for an in vitro diagnostic kit capable of early diagnosis of various diseases (cancer, heart disease, COVID-19, malaria, etc.).

According to an embodiment, the diagnostic kit may use a lateral flow assay (LFA) system.

In the LFA system, the quantum dot-polymer composite may function as a labeling material.

According to an embodiment, the diagnostic kit may use an enzyme-linked immunosorbent assay.

According to another aspect, provided is an immunodetection conjugate including, a quantum dot-polymer composite; and an antibody conjugated to a surface of the quantum dot-polymer composite and specifically binding to a target specimen,
wherein the quantum dot-polymer composite includes one or more quantum dots that has a core-shell structure, a surface-modifying ligand arranged on the surface of the quantum dots, and a quantum-bead of a 3D self-assembly structure including one or more polymer ligands, wherein the one or more quantum dots are dispersed within the quantum-bead, wherein the core includes indium phosphide (InP), and wherein the shell has a thickness of 3 nm to 9 nm.

The terms or elements referred to in the immunodetection conjugate are the same as those referred to in the description of the quantum dot-polymer composite, as described above.

As used herein, the term "immunodetection conjugate" may broadly refer to a specific material and a label conjugated thereto. In the present disclosure, the specific substance may refer to an antibody in an immunodetection conjugate as a substance capable of detecting a specimen in a sample through specific binding, and the label may refer to a quantum dot-polymer composite in an immunodetection conjugate. As used herein, "specific binding" refers to specificity derived from the antibody, which may refer to preferential binding to a defined target specimen or target substance.

As used herein, the term "antibody" may refer to a complete polyclonal or monoclonal antibody, as well as fragments thereof (for example, Fab, Fab', F(ab')2, Fv), single chain (ScFv), diabodies, nanobodies, multispecific antibodies formed from antibody fragments, mutations thereof, fusion proteins including antibody portion, and any other modified alignment of immunoglobulin molecules including antibody recognition sites of the required specificity. Antibodies may include antibodies of any category, for example IgG, IgA, or IgM (or subclasses thereof), and antibodies that do not belong to any specific class.

As used herein, the term "target specimen" may refer to a molecule or other substance in a sample to be detected. For example, the target specimen may include antigenic material, ligands (mono- or polyepitope), haptens, antibodies, and combinations thereof. Furthermore, the target specimen may include, but is not necessarily limited to, toxins, organic compounds, proteins, peptides, microorganisms, amino acids, nucleic acids, hormones, steroids, vitamins, drugs, drug intermediates or by-products, bacteria, virus particles, yeasts, fungi, protozoa, and metabolites of the foregoing substances or antibodies thereto. Preferably, the target specimen is, for example, ferritin; creatinine kinase MB (CK-MB); digoxin; phenytoin; phenobarbitol; carbamazepine; vancomycin; gentamicin; theophylline; valproic acid; quinidine; luteinizing hormone (LH); follicle stimulating hormone (FSH); estradiol, progesterone; C-reactive protein (CRP); lipocalin; IgE antibodies; cytokines; vitamin B2 micro-globulin; glycated hemoglobin (Gly. Hb); cortisol; digitoxin; N-acetylprocainamide (NAPA); procainamide; antibodies to rubella, for example rubella-IgG and rubella IgM; antibodies to toxoplasmosis, for example toxo-IgG and toxoplasmosis IgM; testosterone; salicylates; acetaminophen; hepatitis B virus surface antigens (HBsAgs); antibodies to hepatitis B core antigens, for example antihepatitis B core antigen IgG and IgM (anti-HBC); Human immunodeficiency viruses 1 and 2 (HIV 1 and 2); human T-cell leukemia viruses 1 and 2 (HTLV); hepatitis Be antigen (HBeAg); antibodies to hepatitis B e antigen (anti-HBe); coronavirus; influenza virus; thyroid stimulating hormone (TSH); thyroxine (T4); total triiodothyronine (total T3); free triiodothyronine (free T3); carcinoembryonic antigen (CEA); lipoproteins, cholesterol, and triglycerides; and alpha-fetoprotein (AFP).

As described above, in the case of gold nanoparticles, which have been typically used as materials for in vitro diagnostic kits, have a problem of poor diagnostic sensitivity, making it difficult to diagnose diseases early. Therefore, attempts have been made to use various types of quantum dot composites as materials for diagnostic kits, but there are limitations in human application due to the inherent properties of quantum dots that are sensitive to moisture and the toxicity of inorganic particles.

The quantum dot-polymer composite according to the present disclosure not only enables application as a diagnostic kit for diagnosing diseases in the human body by using biocompatible quantum dots including biocompatible indium phosphide (InP) as a core and not including elements known to be toxic such as cadmium (Cd), lead (Pb), and the like, but also enables excellent photo stability and quantum efficiency by having a unique structure, thereby maximizing luminescence intensity and antigen-antibody binding power, thereby offering enhanced performance.

According to an embodiment, an antibody that specifically binds to the target specimen may be conjugated to the surface of the quantum dot-polymer composite via an isopeptide bond between a first peptide tag and a second peptide tag.

For example, the first peptide tag and the second peptide tag may be one of: a pair of SpyTag and SpyCatcher; a pair of SnoopTag or SnoopTagJr and SnoopCatcher; a pair of RrgATag, RrgATag2 or DogTag and RrgACatcher, a pair of IsopepTag and Pilin-C; a pair of IsopepTag-N and Pilin-N; a pair of PsCsTag and PsCsCatcher; and a pair of SnoopTagJr and DogTag mediated by SnoopLigase, preferably, the first peptide tag and the second peptide tag may be a pair of SpyTag and SpyCatcher.

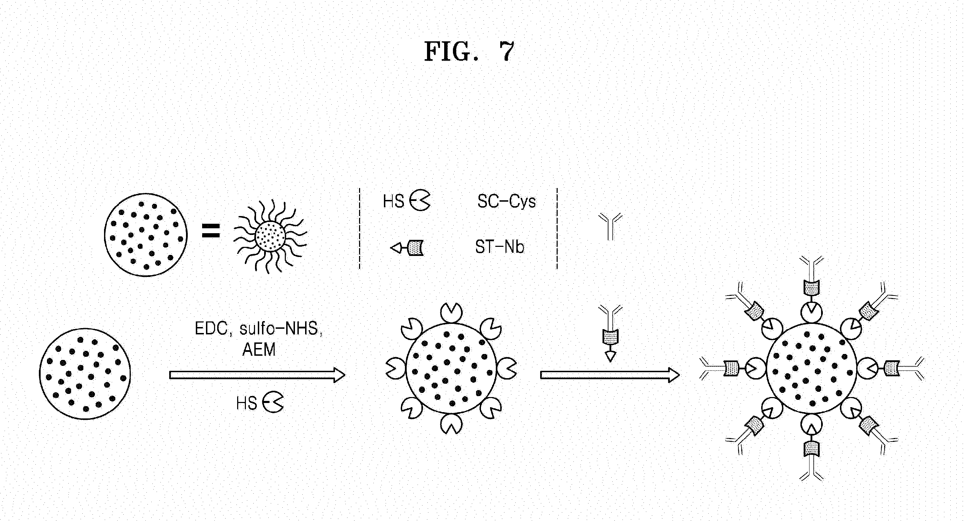

Specifically, referring to FIG. 7, the first tag may be conjugated to the surface of a quantum dot-polymer composite, and the second tag may be conjugated with an antibody that specifically binds to a target specimen. To this end, the first tag may include at least 1 or more cysteine (Cys) in its terminal region (SC-Cys), and the second tag may include a molecule in its terminal region that binds to the FC region of an antibody that specifically binds to the target specimen (ST-Nb). The molecule that binds to the FC region of the antibody, while not forming a bond with the target specimen, may be functionalized to form a specific bond to the FC region of the antibody that specifically binds to the target specimen, thereby exposing the Fab region of the antibody to the outside. The molecule may be, for example, another antibody or fragment thereof having the aforementioned specific binding may be applied, but is not limited thereto.

Here, the terminal region of the first tag, being a region excluding the region forming a bond with the second tag, may be for conjugation with a surface-modifying ligand in the quantum dot-polymer composite, specifically, for forming a disulfide bond with a thiol group of the surface-modifying ligand.

Furthermore, the terminal region of the second tag, being a region excluding the region forming a bond with the first tag, may include a molecule that binds to the FC region of the antibody, thereby allowing the antibody that has a Y-shaped structure to be more densely conjugated to the surface of the quantum dot-polymer composite, as well as allowing the orientation of the antibody to be adjusted such that the Fab region is exposed to the outside. Such conjugation structure between the quantum dot-polymer composite and the antibody may ultimately contribute to improving the detection sensitivity of the target specimen.

According to another aspect, provided is a detection kit for a target specimen, including the immunodetection conjugate.

Terms or elements referred to in the detection kit for a target specimen are the same as those referred to in the description of the immunodetection conjugate, as described above.

The detection kit for a target specimen may be used interchangeably with the terms "diagnostic kit", "discrimination kit", "test kit", depending on the function and use of the target specimen, and the detection kit for a target specimen may be used in a variation of the form employing the technical configuration known in the art, provided that it includes as one component an immunodetection conjugate for the detection and labeling of the target specimen.

According to an embodiment, the detection kit for a target specimen is for detecting an antigen applicable to an antigen-antibody system, and may be an in vitro diagnostic kit based on the antigen. For example, the in vitro diagnostic kit may be for the early diagnosis of various diseases (cancer, heart disease, infectious diseases such as COVID-19, malaria, etc.).

According to an embodiment, the detection kit 220 for a target specimen may be implemented in the form of a lateral flow assay (LFA) strip.

For example, the lateral flow assay strip 220 may include a conjugate pad 222 including the immunodetection conjugate; and a detection portion 230 including a test region 231 immobilized with a second antibody that specifically binds to a target specimen, and a control region 232 immobilized with a third antibody that does not specifically bind to the target specimen but binds to a first antibody that specifically binds to a target specimen in the immunodetection conjugate,

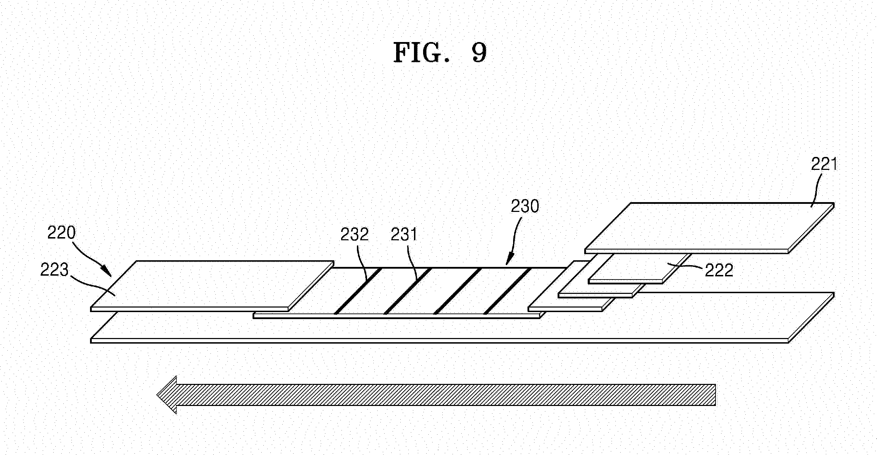

For example, referring to FIG. 9, the lateral flow assay strip 220 may include an absorbent sample pad 221, a conjugate pad 222, and a detection portion 230. The absorbent sample pad 221 may absorb a sample (test solution), and the absorbent sample pad 221 may be manufactured by drying so that the sample can be well absorbed. The conjugate pad 222 is located on a surface below or adjacent the absorbent sample pad 221 and may include an immunodetection conjugate that reacts with the target specimen. The sample absorbed from the absorbent sample pad 221 is transferred to the conjugate pad 222, and if the target specimen is present in the sample, the reaction of the immunodetection conjugate in the conjugate pad 222, specifically an antigen-antibody reaction, results in the formation of an immune composite. The detection portion 230 may include a test region or test line 231 coated with a material capable of binding to the formed immune composite, and a control region or control line 232 coated with a raw material used for control. For example, the control line 232 may include a visible band, and the test line 231 may be one that may be changed to a visible band by the reaction. The detection portion 230 may also be referred to as a membrane, and the material may be made of nitrocellulose, but other materials may be used as needed.

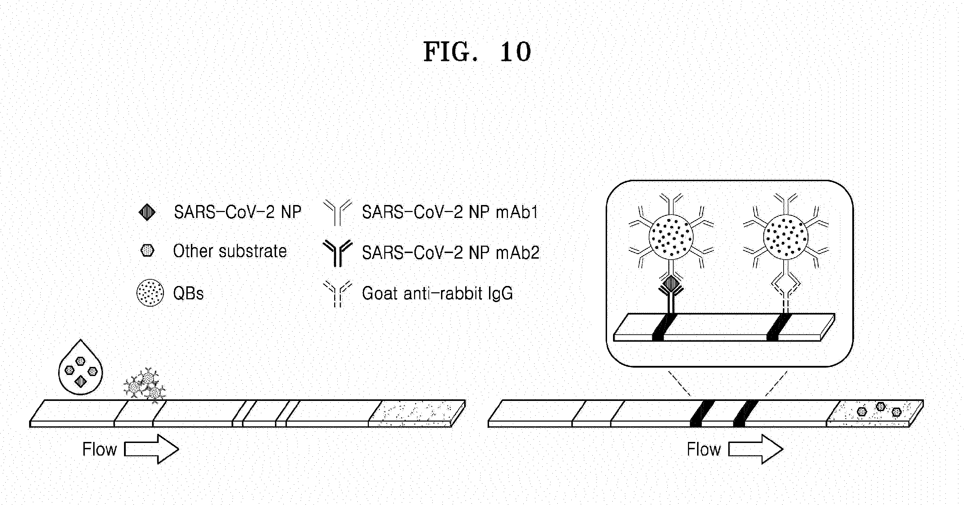

For example, referring to FIG. 10, in the test line 231 of the detection portion 230, a second antibody immobilized on the test line may form a bond with a [target specimen (antigen)-immunodetection conjugate including a first antibody]. Furthermore, at the control line 231 of the detection portion 230, the third antibody immobilized on the control line may form a bond with the remaining immunodetection conjugate that has not formed an immune composite at the conjugate pad 222. Thus, the aforementioned immunodetection conjugate-based antigen-antibody reaction may not only confirm the presence or absence of the target specimen, but may also quantitatively assess the level of the target specimen, if needed.

For example, in the lateral flow assay strip 220, the absorbent sample pad 221, conjugate pad 222, and detection portion 230 may be sequentially arranged in a direction from one end to the other end. Specifically, the absorbent sample pad 221, the conjugate pad 222, and the detection portion 230 may be arranged such that, from one side, the absorbent sample pad 221, the conjugate pad 222, and the detection portion 230 are bound to overlap or is adjacent to each other.

For example, the lateral flow assay strip 220 may further include a hygroscopic pad 223 capable of absorbing unreacted sample from the conjugate pad 222 and the detection portion 230. The hygroscopic pad 223 may absorb a sample that has not reacted between the conjugate pad 222 and the detection portion 230 while coming into contact with a region adjacent to the control line provided in the detection portion 230. The hygroscopic pad 223 may be made of, but not limited to, a cellulose material, and the hygroscopic pad 223 may be manufactured by drying so that the sample can be well absorbed.

According to another aspect, provided is a method of detecting a target specimen, including the process of contacting a sample including the target specimen with the immunodetection conjugate.

Terms or elements referred to in the method of detecting a target specimen are the same as those referred to in the description of an immunodetection conjugate, or a detection kit for a target specimen, as defined above.

As used herein, the term "sample" is not particularly limited, so long as it can contain the target specimen to be detected. For example, the sample may be a biological sample, for example a biological fluid or biological tissue. Examples of biological fluids include urine, blood (whole blood), plasma, serum, saliva, semen, feces, sputum, cerebrospinal fluid, tears, mucus, amniotic fluid, and the like. Biological tissue is an aggregation of cells, generally intracellular substances that form one of the structural substances of humans, animals, plants, bacteria, fungi, or viral structures, and specific types of assemblages, such as connective tissue, epithelial tissue, muscle tissue, and nervous tissue, etc. may correspond to this. Examples of biological tissue may also include organs, tumors, lymph nodes, arteries, and individual cell(s).

As used herein, the term "contact" refers to direct contact between two materials, but it is broadly understood that any additional components may be present, as long as contact is made between the components and the liquid flow.

The following synthesis example and evaluative examples further illustrate the present disclosure. However, the synthesis examples are intended to illustrate the present disclosure and are not intended to limit the scope of the present disclosure.

### I. Synthesis and characterization of quantum dot-polymer composites

### Synthesis Example 1 (Synthesis of indium phosphide (InP) quantum dot (OD) with general shell thickness)

0.8 mmol of InP, 1.6 ml of trioctylphosphine, 3.6 mmol of zinc (5.97 ml of a solution of zinc oleate dissolved in trioctylamine (TOA)), and 0.5 mmol of selenide (0.25 ml of a solution of selenide dissolved in trioctylphosphine) were placed in a 3-neck flask and degassed using a vacuum pump.

After purging with argon, the reaction was heated to 180 °C under an argon gas atmosphere, and 0.15 ml of a mixed solution of hydrofluoric acid-trioctylamine with a volume ratio of 1:1 was injected to proceed with the reaction for 30 minutes.

The temperature was then raised to 340 °C and 1.4 mmol of zinc (2.33 ml of a solution of zinc oleate dissolved in trioctylamine) was injected once and 0.5 mmol of selenide (0.25 ml of a solution of selenide dissolved in trioctylphosphine) was injected once to form a ZnSe shell, and to form a ZnS shell the temperature was lowered to 320 °C and 1.2 mmol of zinc (2.0 ml of a solution of zinc oleate dissolved in trioctylamine) and 0.25 mmol of sulfur (0.125 ml of a solution of sulfur dissolved in trioctylphosphine) were alternately injected once and twice, respectively, and the synthesis was terminated.

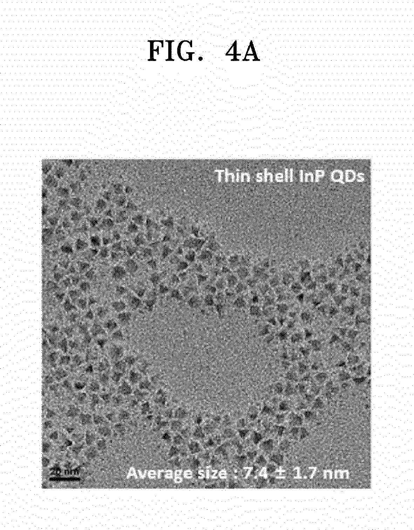

The TEM image of the above quantum dot is shown in FIG. 4A, and the shell thickness was calculated by considering the diameter of the InP nanoparticles (3 nm) from the size of the above quantum dot, and it was found that the shell thickness was about 2.2 nm.

### Synthesis Example 2 (Synthesis of InP QD with thick shells)

0.8 mmol of InP, 1.6 ml of trioctylphosphine, 3.6 mmol of zinc (5.97 ml of a solution of zinc oleate dissolved in trioctylamine), and 0.5 mmol of selenide (0.25 ml of a solution of selenide dissolved in trioctylphosphine) were placed in a 3-necked flask and degassed using a vacuum pump.

After purging with argon, the reaction was heated to 180 °C under an argon gas atmosphere, and 0.15 ml of a mixed solution of hydrofluoric acid-trioctylamine with a volume ratio of 1:1 was injected to proceed with the reaction for 30 minutes.

The temperature was then raised to 340 °C and 1.4 mmol of zinc (2.33 ml of a solution of zinc oleate dissolved in trioctylamine) was injected once and 0.5 mmol of selenide (0.25 ml of a solution of selenide dissolved in trioctylphosphine) was injected twice to form a thick ZnSe shell, and the temperature was lowered to 320 °C for ZnS shell formation and 1.2 mmol of zinc (2.0 ml of solution of zinc oleate dissolved in trioctylamine) and 0.25 mmol of sulfur (0.125 ml of a solution of sulfur dissolved in trioctylphosphine) were alternately injected twice and eight times, respectively.

Then, 0.5 mmol of sulfur (0.25 ml of a solution of sulfur dissolved in trioctylphosphine) was injected once more and the reaction was maintained at 260 °C for 1 hour, and the temperature was lowered to 180 °C and 0.15 ml of 1-dodecanethiol was injected to add the ligand to the surface of the quantum dots and the synthesis was terminated.

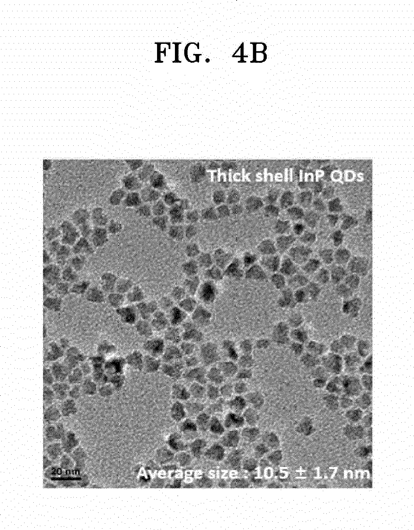

The TEM image of the above quantum dot is shown in FIG. 4B, and the shell thickness was calculated by considering the diameter of the InP nanoparticles (3 nm) from the size of the above quantum dot, and it was found that the shell thickness was about 3.75 nm.

### (Manufacture of quantum-bead)

### Example 1

Quantum dots (0.6 mg) and polystyrene-polyacrylic acid block copolymer (4 mg) (polymer ligand) synthesized in Synthesis Example 2 were dispersed in tetrahydrofuran (1 ml) and water (1 ml) was added under stirring to manufacture a quantum-bead.

### Comparative Example 1

Quantum dots (0.6 mg) and polystyrene-polyacrylic acid block copolymer (4 mg) (polymer ligand) synthesized in Synthesis Example 1 were dispersed in tetrahydrofuran (1 ml) and water (1 ml) was added under stirring, and then purified to manufacture a quantum-bead.

### Example 1 (Confirmation of quantum dot dispersion in quantum-bead)

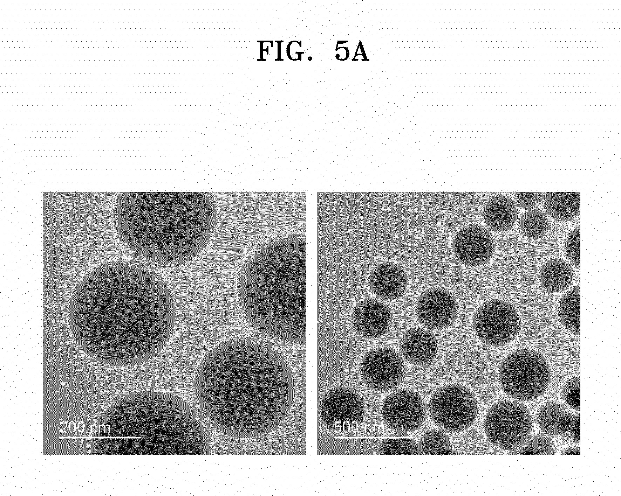

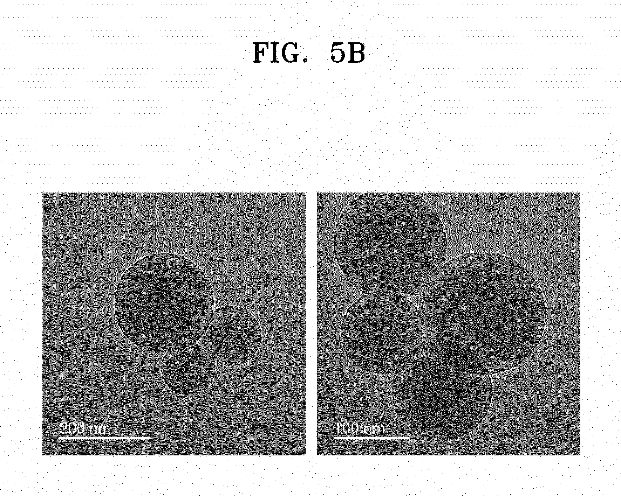

TEM images of the quantum-bead manufactured in Example 1 and Comparative Example 1 were obtained and shown in FIG. 5A and FIG. 5B, respectively.

As shown in FIG. 5A and FIG. 5B, it is confirmed that the quantum-bead of Example 1 have a greater amount of uniformly dispersed quantum dots compared to the quantum-bead of Comparative Example 1. Proving that the quantum dots with thicker shells used in Example 1 not only have better interaction with the polymer during quantum-bead formation, but also that the stability of the quantum dots to the solvent is superior compared to the quantum dots with thinner shells used in Comparative Example 1.

### Example 2 (Confirmation of phase change stability of quantum-bead)

To confirm the phase change stability of the quantum-bead manufactured in Example 1 and Comparative Example 1, the quantum dots obtained in Synthesis Example 1 and Synthesis Example 2 and the quantum-bead obtained in Example 1 and Comparative Example 1 were each added to a test tube including a hexane/water mixture.

The test tubes were then irradiated with UV light to measure the emitted light from the quantum dots, respectively, and the optical efficiency of the quantum dots of Synthesis Example 2 relative to the optical efficiency of the quantum-bead of Example 1 and Example 2 was calculated and graphed, and the test tubes were visually observed.

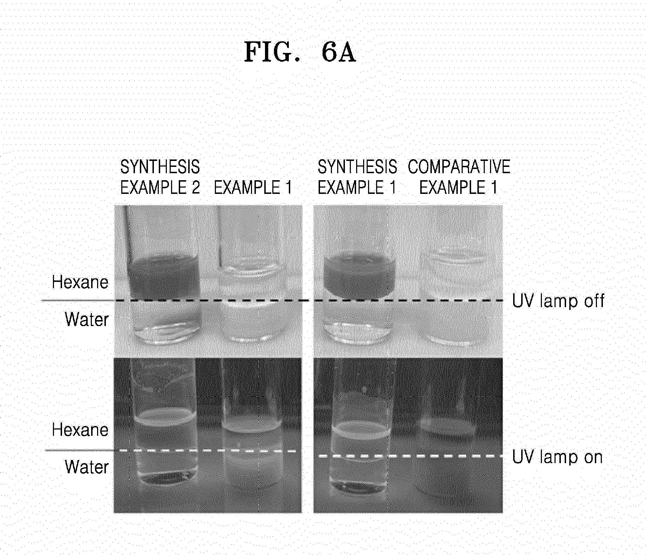

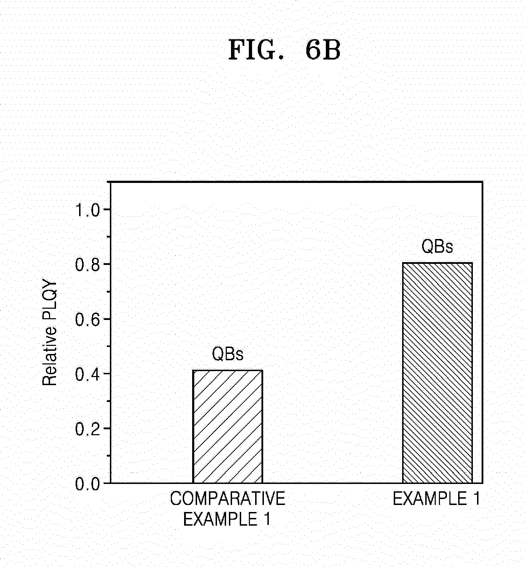

A picture of each test tube can be seen in FIG. 6A, and a graph of the optical efficiency can be seen in FIG. 6B.

As shown in FIG. 6A and FIG. 6B, it may be seen that when InP quantum dots with a thin shell thickness are manufactured into a quantum-bead, the phase transition to aqueous phase is successfully achieved, but the luminous efficiency is significantly reduced. This is due to the degradation of the InP quantum dots during the modification into the quantum-bead due to the thin shell thickness and the inability to sufficiently disperse within the bead.

### II. Manufacture of immunodetection conjugate and evaluation of its functionality

In this embodiment, using the quantum-bead manufactured above, an immunodetection conjugate that has an antibody conjugated to the surface of the quantum-bead was manufactured and its functionality was evaluated. Specifically, an immunodetection conjugate in which the orientation of the antibody is adjusted so that the Fab region of the antibody is exposed to the outside was manufactured, and the detection efficacy of a lateral flow assay strip including the immunodetection conjugate was evaluated.

### Example 2. Manufacture and functional evaluation of immunodetection conjugate

### (2.1) Manufacture of immunodetection conjugate

Antibodies used for the detection of target substances were conjugated to the surface of the quantum-bead (QB) manufactured in Example 1 above by a covalent bond coupling method. Specifically, water-soluble quantum-bead (0.1 mg), EDC (59.94 µg), and sulfo-NHS (135.80 µg) were mixed in 200 µL of MES buffer (0.01 M, pH 6.0), wherein the molar ratio of EDC and sulfo-NHS was adjusted to 1:2. The mixture was then activated in a thermomixer (25 °C, 300 rpm) for 30 minutes, after which the quantum-beads were separated by centrifugation and redispersed in 200 µL of PBS (0.01 M, pH 8.0). Then, anti-hCG mAb1 (25 µg) was added to the above quantum-bead and mixed for 3 hours. The synthesized immunodetection conjugate was purified by centrifugation (13,000 rpm, 10 minutes) and 200 µL of blocking buffer (1x PBS containing 0.1% (w/v) Tween-20 and 3% (w/v) BSA) was added, and reacted for 30 minutes to block further reaction on the quantum-bead surface. The reaction-blocked immunodetection conjugate was centrifuged (13,000 rpm, 10 minutes), the supernatant was discarded, and the pelleted immunodetection conjugate was resuspended in 100 µL of the above blocking buffer.

### (2.2) Manufacture of immunodetection conjugate with adjusted antibody orientation

An immunodetection conjugate in which the orientation of the antibody is adjusted to expose the Fab region to the outside was manufactured by adopting the synthesis process of the immunodetection conjugate described above, as shown in FIG. 7, but adding the process of introducing Fc region-specifically binding nanobody (Nb). As the Fc region-specifically binding nanobody, a camelidae-derived a single monomeric variable Ab domain designed to specifically capture/bind the FC region of an IgG1 antibody was used. Specifically, quantum-bead (QB), EDC (59.94 µg), suflo-NHS (135.80 µg), and AEM (276.11 µg) manufactured from Example 1 were mixed in 200 µL of 1× PBS (0.01 M, pH 7.4). After 1 hour of activation, the mixture was centrifuged (13,000 rpm, 10 minutes) to remove residual reaction reagents. To perform the coating of nanobodies on the pelletized quantum-bead, SC-Cys (SpyCatcher-Cys, 43.48 µg) and ST-Nb (SpyTag-Nb, 52.21 µg) were added sequentially. After reaction for 12 hours, the Nb-coated quantum-bead were centrifuged (13,000 rpm, 10 minutes), washed, and redispersed in 200 µL of 1× PBS (pH 7.4). Then, anti-hCG mAb1 was added for final conjugation, and the subsequent processes were performed in the same manner as in Example (2.1) above.

### (2.3) Quantification of antibodies conjugated to quantum-bead and evaluation of Fab region accessibility

The number of antibodies conjugated to the quantum-bead was calculated as the difference between ma (the amount of antibody added) and mr (the amount of antibody remaining in the supernatant after addition of antibody to the activated quantum-bead). mr was calculated by multiplying the concentration of antibodies by the total volume. The BCA assay was used to calculate the concentration of antibodies, and the BCA calibration curve was determined by measuring the absorbance at 562 nm of a BSA protein standard substance (0-200 µg mL-1) after incubation at 60 °C for 30 minutes. In addition, to assess the accessibility of Fab regions, i.e., the degree of exposure to the outside, immunodetection conjugate (QB-mAb1, 50 µL) in blocking buffer and anti-mouse Fab region goat antibody-Alexa 647 (50 µL) in 1 x PBS (0.01 M, pH 7.4) were mixed and bound for 1 hour, followed by centrifugation (13,000 rpm, 10 minutes). The difference between the amount added and the amount retained of anti-mouse Fab region goat Ab-Alexa 647 in the supernatant, which is considered to be the number of accessible Fab regions, was calculated by measuring the absorbance at 652 nm.

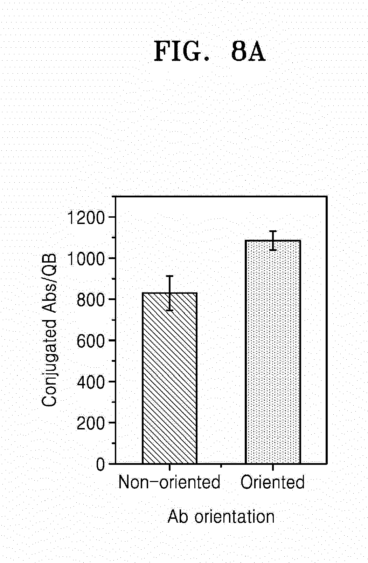

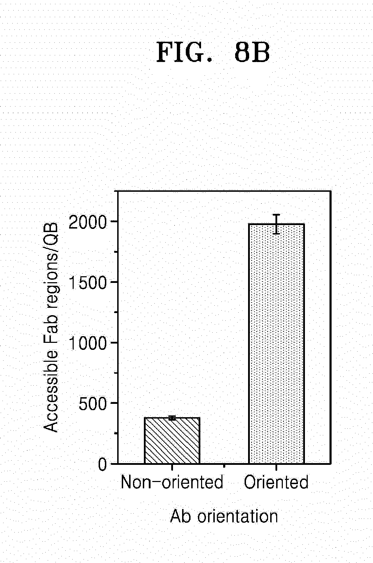

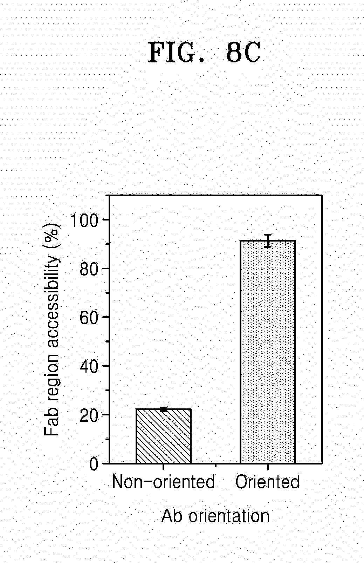

As a result, as shown in FIG. 8A, it was found that the immunodetection conjugate according to an embodiment were successfully synthesized, and in particular, in the case of the immunodetection conjugate of embodiment (2.2), it was found that by controlling the orientation of the conjugated antibody that has a Y-shaped structure, a more dense conjugation of the antibody to the surface of the quantum-bead could be achieved. Furthermore, as shown in FIG. 8B and FIG. 8C, the immunodetection conjugate of Example 2.2 in which the orientation of the antibody was adjusted was confirmed to have an accessible Fab region/QB value and an access rate (%) to the Fab region about 5 times higher compared to the immunodetection conjugate of Example 2.1.

The above results suggest that through the sequential chemical conjugation and mixing process described in Example 2.2, an immunodetection conjugate with a controlled orientation of the antibody (QB-SC-ST-Nb-mAb1) may be successfully manufactured, thereby improving the detection efficacy.

### Example 3. Evaluation of detection efficacy of lateral flow assay strip on target specimen

In this embodiment, the above orientation adjusted immunodetection conjugate was used to manufacture a lateral flow assay strip for detecting SARS-coV-2 NPs as a target specimen. For this purpose, a membrane (Nupore), a sample pad (Milipore), a conjugate pad (Boreda biotech), an absorbent pad (Boreda biotech), and a laminated card (Boreda biotech) were obtained. The membrane was then attached to a 30 cm long laminated card, and the test line (concentration 1.5 mg/mL) and control line (concentration 0.5 mg/mL) were formed on the membrane, which were then dried at 37 °C for 2 hours. The dried membrane was then immersed in blocking buffer (1 XPBS containing 2% BSA) for 20 minutes, and then the blocked membrane was immersed in washing solution (1 XPBS containing 0.05% sodium dodecyl sulfate) for another 30 minutes. The washed membrane was dried in an oven at 37 °C for 2 hours, and then a pre-prepared sample pad, a conjugate pad, and an absorbent pad were attached to the membrane, and each of them were cut into a size of 4 mm. Meanwhile, the conjugate pad was manufactured by dispensing a mixture of 1.2 µL of the above immunodetection conjugate and 10.8 µL of dilution buffer (1x PBS containing 0.1% Tween-20, 1% BSA, and 10% sucrose) onto the conjugate pad, and then drying in a vacuum oven at room temperature for 30 minutes.

Then, 80 µL of the sample solution manufactured by diluting the SARS-CoV-2 NP storage solution with 1X PBS was dropped onto the sample pad of the manufactured lateral flow assay strip and allowed to pass through the membrane by capillary action. The concentrations of SARS-CoV-2 NPs in the above sample solutions were adjusted to 200 ng/ml, 20 ng/ml, 2 ng/ml, 200 pg/ml, 100 pg/ml, 50 pg/ml, and 20 pg/ml, respectively. All solutions were absorbed by the absorbent pads within 20 minutes, and the results were measured using a portable UV lamp (UVITec LF-206.LS) and a Samsung Galaxy S20 smartphone were used to measure and record the results. The images were then scanned and quantitative assay were performed by measuring the fluorescence intensity with an AXIO ZOOM stereo microscope (Zeiss).

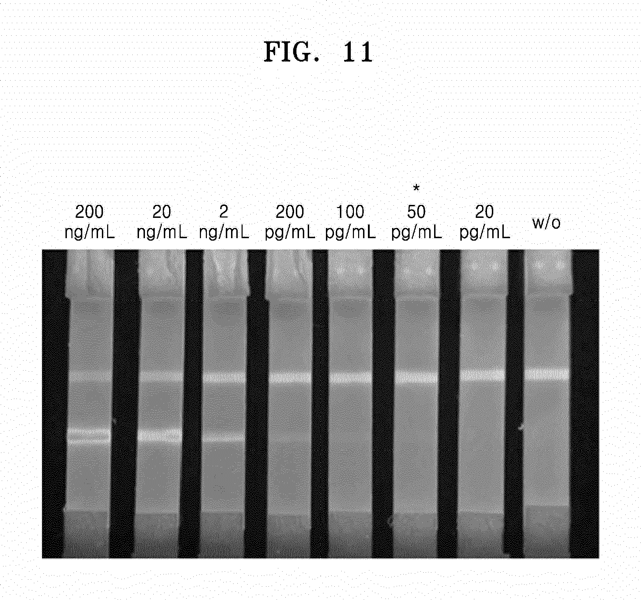

As a result, as shown in FIG. 11, a lateral flow assay strip including an immunodetection conjugate according to an embodiment may exhibit valid detection efficacy even in a sample including a target specimen at a level of 50 pg/mL.

## Claims

1. A quantum dot-polymer composite, comprising a quantum-bead of a 3D self-assembly structure comprising one or more quantum dots that has a core-shell structure, a surface-modifying ligand arranged on the surface of the quantum dots, and one or more polymer ligands,
wherein the one or more quantum dots are dispersed within the quantum-bead,
wherein the core comprises indium phosphide (InP),
wherein the shell has a thickness of from 3 nm to 9 nm.

2. The quantum dot-polymer composite of claim 1, wherein the shell has a thickness of 3 nm to 5 nm.

3. The quantum dot-polymer composite of claim 1, wherein the shell comprises one or more inorganic particles of zinc telluride (ZnTe), zinc sulfide (ZnS), zinc oxide (ZnO), zinc selenide (ZnSe), zinc selenide sulfide (ZnSeS), indium gallium nitride (InGaN), indium gallium phosphide (InGaP), indium zinc phosphide (InZnP), copper selenide telluride (CuSeTe), copper indium selenide (CuInSe₂), copper indium sulfide (CuInS₂), silver indium sulfide (AgInS₂), and tin telluride (SnTe).

4. The quantum dot-polymer composite of claim 3, wherein the shell comprises 2 or more different types of inorganic particles.

5. The quantum dot-polymer composite of claim 4, wherein the shell comprises a first shell layer and a second shell layer, and
the first shell layer comprises a first inorganic particle and the second shell layer comprises a second inorganic particle.

6. The quantum dot-polymer composite of claim 5, wherein the first shell layer is arranged between a core of the quantum dot and the second shell layer, and wherein a band gap of the second inorganic particle is greater than a band gap of the first inorganic particle.

7. The quantum dot-polymer composite of claim 1, wherein, when the number of one or more quantum dots is two or more, the two or more quantum dots are identical or different from each other.

8. The quantum dot-polymer composite of claim 1, wherein the surface-modifying ligand comprises one or more thiol groups.

9. The quantum dot-polymer composite of claim 1, wherein the surface-modifying ligand is derived from a linear, branched, or aromatic compound comprising one or more thiol groups as functional groups.

10. The quantum dot-polymer composite of claim 1, wherein the surface-modifying ligand is a C₁-C₂₀ alkylthiol group comprising one or more thiol groups.

11. The quantum dot-polymer composite of claim 1, wherein the one or more polymer ligands are selected from a random copolymer, a block copolymer, a graft copolymer, or a combination thereof.

12. The quantum dot-polymer composite of claim 1, wherein the one or more polymer ligands each comprise a hydrophobic portion and a hydrophilic portion.

13. The quantum dot-polymer composite of claim 12, wherein the hydrophobic portion of the one or more polymer ligands is aligned toward a center direction of the quantum-bead, and the hydrophilic portion of the one or more polymer ligands is aligned toward the outside direction of the quantum-bead.

14. The quantum dot-polymer composite of claim 12, wherein the hydrophilic portion of the one or more polymer ligands is in the form of a needle or in the form of a brush.

15. The quantum dot-polymer composite of claim 1, wherein the one or more polymer ligands has an end including one or more of an amine group, an amide group, a hydroxy group, a maleimide group, a thiol group, and a carboxylic acid group.

16. The quantum dot-polymer composite of claim 1, wherein the one or more polymer ligands comprise one or more selected from polystyrene, polyolefin, polyvinyl acetal, polyester, polyketone, polyether, polyvinyl pyridine, polycarbonate, polydiene, polyacrylic acid, polyamide, and polyimide; random copolymers and block copolymers comprising a poly maleic anhydride group and an alkylamine.

17. The quantum dot-polymer composite of claim 16, wherein the polymer comprising the poly maleic anhydride group is represented by Formula 1 below, and the graft copolymer comprising the poly maleic anhydride group and the alkylamine is represented by Formula 1A or Formula 1B below: 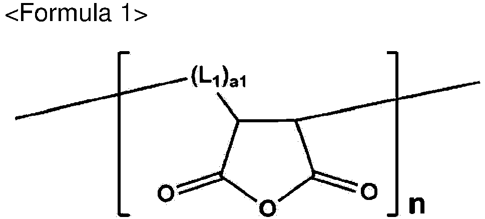 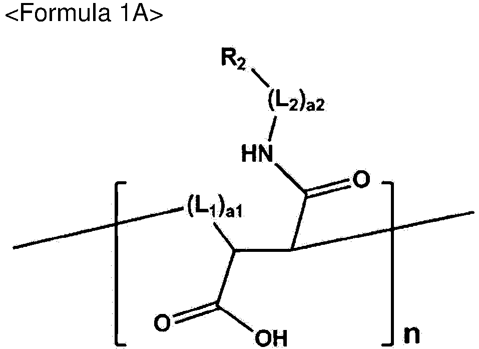 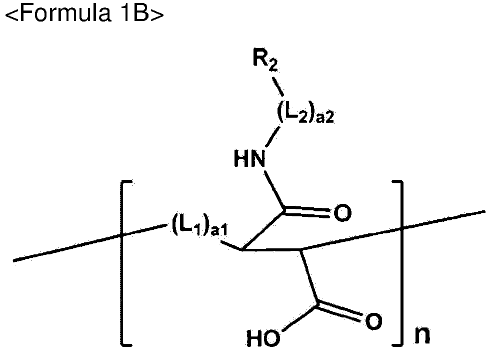
wherein, in Formula 1, Formula 1A, and Formula 1B,
L₁ and L₂ are each independently selected from: a C₁-C₂₀ alkylene group; and a C₁-C₂₀ alkylene group substituted with one or more selected from deuterium, -F, -Cl, -Br, -I, a cyano group, a nitro group, a C₁-C₁₀ alkyl group, a C₆-C₂₀ aryl group, and a C₁-C₁₀ alkoxy group,
a1 is an integer from 1 to 5,
a2 is an integer from 0 to 5,
R₂ is selected from a C₁-C₂₀ alkyl group; and a C₁-C₂₀ alkyl group substituted with one or more selected from deuterium, -F, -Cl, -Br, -I, a cyano group, a nitro group, a C₁-C₁₀ alkyl group, a C₆-C₂₀ aryl group, and a C₁-C₁₀ alkoxy group,
n is at least 1 but not more than 1000.

18. The quantum dot-polymer composite of claim 1, wherein a quantum-bead has a hydrodynamic diameter (HD) of 50 nm to 1000 nm.

19. A diagnostic kit, comprising a quantum dot-polymer composite according to any one of claims 1 to 18.

20. The diagnostic kit of claim 19, wherein the diagnostic kit uses a lateral flow assay (LFA) system.

21. An immunodetection conjugate, comprising:
a quantum dot-polymer composite; and
an antibody conjugated to a surface of the quantum dot-polymer composite and specifically binding to a target specimen,
wherein the quantum dot-polymer composite comprises a quantum-bead of a 3D self-assembly structure which comprises: one or more quantum dots that has a core-shell structure; a surface-modifying ligand arranged on the surface of the quantum dot; and one or more polymer ligands,
wherein the one or more quantum dots are dispersed within the quantum-bead,
wherein the core comprises indium phosphide (InP),
wherein the shell has a thickness of 3 nm to 9 nm.

22. The immunodetection conjugate of claim 21, wherein the shell comprises one or more inorganic particles of zinc telluride (ZnTe), zinc sulfide (ZnS), zinc oxide (ZnO), zinc selenide (ZnSe), zinc selenide sulfide (ZnSeS), indium gallium nitride (InGaN), indium gallium phosphide (InGaP), and indium zinc phosphide (InZnP), copper selenide telluride (CuSeTe), copper selenide (CuInSe₂), copper indium sulfide (CuInS₂), silver indium sulfide (AgInS₂), and tin telluride (SnTe).

23. The immunodetection conjugate of claim 22, wherein the shell comprises 2 or more different types of inorganic particles.

24. The immunodetection conjugate of claim 23, wherein the shell comprises a first shell layer and a second shell layer,
wherein the first shell layer comprises a first inorganic particle and the second shell layer comprises a second inorganic particle.

25. The immunodetection conjugate of claim 24, wherein the first shell layer is arranged between a core of the quantum dot and the second shell layer, and wherein a band gap of the second inorganic particle is greater than a band gap of the first inorganic particle.

26. The immunodetection conjugate of claim 21, wherein the surface-modifying ligand comprises one or more thiol groups.

27. The immunodetection conjugate of claim 21, wherein the one or more polymer ligands are selected from a random copolymer, a block copolymer, a graft copolymer, or a combination thereof.

28. The immunodetection conjugate of claim 21, wherein each of the one or more polymer ligands has an end comprising one or more of an amine group, an amide group, a hydroxy group, a maleimide group, a thiol group, and a carboxylic acid group.

29. The immunodetection conjugate of claim 21, wherein the one or more polymer ligands comprise one or more selected from polystyrene, polyolefin, polyvinyl acetal, polyester, polyketone, polyether, polyvinyl pyridine, polycarbonate, polydiene, polyacrylic acid, polyamide, and polyimide; random copolymers and block copolymers thereof; polymers comprising a poly maleic anhydride group; and graft copolymers comprising a poly maleic anhydride group and an alkylamine.

30. The immunodetection conjugate of claim 21, wherein the quantum-bead has a hydrodynamic diameter (HD) of 50 nm to 1000 nm.

31. The immunodetection conjugate of claim 21, wherein the antibody that specifically binds to the target specimen is conjugated to a surface of the quantum dot-polymer composite via an isopeptide bond between a first peptide tag and a second peptide tag.

32. The immunodetection conjugate of claim 31, wherein the first peptide tag and second peptide tag are one of: a pair of SpyTag and SpyCatcher; a pair of SnoopTag or SnoopTagJr and SnoopCatcher; a pair of RrgATag, RrgATag2 or DogTag and RrgACatcher, a pair of IsopepTag and Pilin-C; a pair of IsopepTag-N and Pilin-N; a pair of PsCsTag and PsCsCatcher; and a pair of SnoopTagJr and DogTag mediated by SnoopLigase.

33. The immunodetection conjugate of claim 31, wherein the first tag comprises a cysteine (Cys) in a terminal region thereof,
wherein the second tag comprises, in a terminal region thereof, a molecule that binds to the FC region of an antibody that specifically binds to the target specimen.

34. A detection kit for a target specimen, comprising an immunodetection conjugate according to any one of claims 21 to 33.

35. The kit of claim 34, wherein the target specimen is one or more of an autoantibody, a ligand, a natural extract, a peptide, a protein, a metal ion, a synthetic drug, a natural drug, a metabolite, a genome, a virus, and a substance produced by a virus, and bacteria and a substance produced by a virus.

36. The kit of claim 35, wherein the kit is a lateral flow assay strip.

37. The kit of claim 36, wherein the lateral flow assay strip comprises:
a conjugate pad comprising an immunodetection conjugate according to any one of claims 21 to 33; and
a detection portion including a test region in which a second antibody is immobilized, the second antibody that specifically binds to a target specimen and a control region in which a third antibody is immobilized, the third antibody that does not specifically bind to the target specimen but binds to a first antibody that specifically binds to a target specimen in the immunodetection conjugate.

38. The kit of claim 37, wherein the lateral flow assay strip further comprises an absorbent sample pad,
wherein the absorbent sample pad, the conjugate pad, and the detection portion are sequentially arranged in a direction from one end to the other end of the lateral flow assay strip.
